# EUROPEAN PATENT SPECIFICATION

(11) **EP 3 368 654 B1**
(45) Date of publication and mention of the grant of the patent: **19.11.2025**
(21) Application number: 16860490.8
(22) Date of filing: 07.10.2016
(51) Int. Cl.: C12M 1/34, C12N 15/09, C12P 19/34, G01N 21/64, C12Q 1/6858, C12Q 1/683

(54) **DETERMINATION OF POLYMORPHISMS USING ISOTHERMAL NUCLEIC ACID AMPLIFICATION**
BESTIMMUNG VON POLYMORPHISMEN MITTELS ISOTHERMER NUKLEINSÄUREAMPLIFIKATION
DÉTERMINATION DES POLYMORPHISMES PAR AMPLIFICATION ISOTHERME D'ACIDE NUCLÉIQUE

(30) Priority: 30.10.2015 US 201562248895 P
(43) Date of publication of application: 05.09.2018
(73) Proprietor: Abbott Diagnostics Scarborough, Inc., Scarborough, ME 04074 (US)
(72) Inventor: FORREST, Matthew Simon, Cambridge Cambridgeshire CB1 3AR (GB); ARMES, Niall A., Helions Bumpstead Suffolk CB9 7AW (GB)
(74) Representative: Mathys & Squire
(86) International application number: PCT/US2016/055949
(87) International publication number: WO 2017/074688

(56) References cited:
- WO-A1-01/49877
- US-A1- 2007 092 883
- US-A1- 2010 311 127
- US-A1- 2012 115 737
- US-A1- 2015 064 138
- US-A1- 2015 104 788
- ZHAO JINYIN ET AL: "Restriction endonuclease-mediated real-time digestion-PCR for somatic mutation detection", INTERNATIONAL JOURNAL OF CANCER, vol. 132, no. 12, June 2013 (2013-06-01), pages 2858 - 2866, XP002790807
- SAIKI ET AL.: "Enzymatic Amplification of beta-Globin Genomic Sequences and Restriction Site Analysis for Diagnosis of Sickle Cell Anemia", SCIENCE, vol. 230, 20 December 1985 (1985-12-20), pages 1350 - 4, XP009017837
- UPTON ET AL.: "Arylamine N-acetyltransferase of Mycobacterium tuberculosis is a polymorphic enzyme and a site of isoniazid metabolism", MOL MICROBIOL, vol. 42, 1 October 2001 (2001-10-01), pages 309 - 17, XP055379955
- KERKEL ET AL.: "Genomic surveys by methylation-sensitive SNP analysis identify sequence-dependent allele-specific DNA methylation", vol. 40, 22 June 2008 (2008-06-22), pages 904 - 908, XP055379958

## Description

### TECHNICAL FIELD

This invention relates to a method for detecting a polymorphism in a target nucleic acid sequence using isothermal nucleic acid amplification. More specifically, the present invention relates to using recombinase polymerase amplification (RPA) to detect single nucleotide polymorphisms in a target nucleic acid sequence.

### BACKGROUND

Certain isothermal amplification methods are able to amplify target nucleic acid from trace levels to very high and detectable levels within a matter of minutes. Such isothermal methods, e.g., Recombinase Polymerase Amplification (RPA), can allow users to detect a particular sequence in trace amounts, facilitating point-of-care testing and increasing the accessibility and speed of diagnostics. Zaoh J. et al., Int. J. Cancer, 132, pp.2858-2866 (2013) and WO0149877 A1 describe methods for detecting genetic polymorphisms by using endonucleases.

Single nucleotide polymorphisms (SNPs) represent an abundant form of genetic variation in humans. SNPs can provide important genetic markers for disease diagnosis or prognosis. Furthermore, the identification of SNPs (and other genetic polymorphisms) can play a significant role in helping to tailor drugs and drug regimens to particular genotypes. As a consequence of the clear impact that pharmacogenetics can, and will, have on the healthcare industry, there is a pressing need to develop improved methods of genotype testing. More particularly, there is a need to develop improved methods for the detection of polymorphisms (e.g., a SNP) in a target nucleic acid sequence that improve the sensitivity and specificity to enable accurate and efficient routine testing procedures.

### SUMMARY

This disclosure is based, at least in part, on the discovery that genetic polymorphisms can be accurately and efficiently detected in target nucleic acid sequences using RPA. In view of this discovery, provided herein are RPA compositions and methods for detecting the presence or absence of a polymorphism in a target nucleic acid. These polymorphisms can be diagnostic of disease or disorder.

In one aspect, not being part of the present invention, this disclosure features compositions that include: (a) a first and second primer for amplifying the target nucleic acid sequence, (b) one or more recombinase(s), (c) one or more polymerase(s), and (d) an agent capable of cleaving double stranded nucleic acid at a target cleavage sequence. In some embodiments, the target cleavage sequence is present in the target nucleic acid sequence. In some embodiments, the target cleavage sequence differs from the target nucleic acid sequence at one or more positions and the first primer is complementary to the target nucleic acid sequence and the second primer comprises a first portion complementary to the target nucleic acid sequence and a second portion of that differs from the target nucleic acid at the one or more positions and consists of at least a portion of the target cleavage sequence.

The compositions include a probe labeled with a detectable label. In some embodiments, the detectable label is a fluorophore, an enzyme, a quencher, an enzyme inhibitor, a radioactive label, an electrochemical label, a chemiluminescent label, a metal sol particle, a latex particle, one member of a binding pair or any combination thereof.

The recombinase(s) include T4 bacteriophage UvsX, T6 bacteriophage UvsX, Rb69 UvsX, Aeh1 UvsX, RecA, T2 bacteriophage UvsX, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, phage LZ2, RADA RADB, Rad51 proteins, or any combination thereof.

The polymerase(s) include *E. coli* DNA polymerase I (e.g., Klenow fragment), bacteriophage T4 gp43 DNA polymerase, *Bacillus stearothermophilus* polymerase I large fragment, Phi-29 DNA polymerase, T7 DNA polymerase, *Bacillus subtilis* Pol I, *Staphylococcus aureus* Pol I, *E. coli* DNA polymerase I, *E. coli* DNA polymerase II, *E. coli* DNA polymerase III, *E. coli* DNA polymerase IV, *E*. *coli* DNA polymerase V, or any combination thereof.

The composition further includes a single stranded DNA binding protein, e.g., *E. coli* SSB and those derived from myoviridae phages, such as T4, T2, T6, Rb69, Aeh1, KVP40, *Acinetobacter* phage 133, *Aeromonas* phage 65, cyanophage P-SSM2, cyanophage PSSM4, cyanophage S-PM2, Rb14, Rb32, Aeromonas phage 25, *Vibrio* phage nt-1, phi-1, Rb16, Rb43, Phage 31, phage 44RR2.8t, Rb49, phage Rb3, or phage LZ2.

The agent capable of cleaving double stranded nucleic acid at a target cleavage sequence is a restriction enzyme or endonuclease, a Zinc finger, a CRISPR-nuclease, or a TALEN. In some embodiments the restriction endonuclease is DdelI or Hpy166II. In some embodiments, the agent is not ExoIII, Fpg or Nfo.

The composition further comprises a crowding agent, e.g., one or more of polyethylene glycol (PEG)(e.g., PEG1450, PEG3000, PEG8000, PEG10000, PEG14000, PEG15000, PEG20000, PEG250000, PEG30000, PEG35000, PEG40000, PEG compound with molecular weight between 15,000 and 20,000 daltons, or combinations thereof), dextran, polyvinyl alcohol, polyvinyl pyrrolidone, and Ficoll. In some embodiments, the crowding agent is present in the reaction mixture at a concentration between 1 to 12% by weight or by volume of the reaction mixture, e.g., between any two concentration values selected from 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, and 12.0%.

The first and second primers consist, comprise, or consist essentially of an oligonucleotide having a length of at least or about 10 nucleotides, at least about 20 nucleotides, at least about 30 nucleotides, at least about 40 nucleotides, or at least 50 nucleotides.

The composition further comprises (1) third and fourth primers and (2) a second agent capable of cleaving a double stranded nucleic acid at a second target cleavage sequence. In some embodiments of all aspects the second target cleavage sequence differs from the "first" target nucleic acid sequence at one or more positions. In some embodiments the third primer is complementary to the target nucleic acid sequence. In some embodiments, a first portion of the fourth primer is complementary to the target nucleic acid sequence and a second portion of the fourth primer comprises at least part of the second target cleavage sequence including at least one of the one or more positions where the second specific cleavage sequence differs from the target nucleic acid sequence. The third and fourth primers can be the same or different from the first and second primers.

In another aspect, this disclosure features methods of determining the presence or absence of a polymorphism in a target nucleic acid sequence that include: (a) contacting the target nucleic acid sequence with a mixture including: a first primer and a second primer for amplifying the target nucleic acid sequence; a recombinase, a polymerase, and an agent capable of cleaving double-stranded nucleic acid at a target cleavage sequence; (b) performing a nucleic acid amplification reaction of the mixture for production of nucleic amplification products in the mixture; and (c) monitoring the rate of increase of nucleic acid amplification products in the mixture; wherein an exponential rate of increase of nucleic amplification products indicates the presence or absence of the polymorphism in the target nucleic acid sequence.

In some embodiments of any of the aspects described here, the polymorphism is a single nucleotide polymorphism (SNP). In some embodiments of all aspects, the nucleic acid amplification reaction is recombinase polymerase amplification (RPA) reaction. In some embodiments of all aspects the monitoring of the rate of increase of nucleic acid amplification products in the mixture is performed in real-time.

In some embodiments of any of the aspects described here, the presence of a polymorphism is determined by the cleavage of the nucleic amplification products with the agent. In some embodiments the target cleavage sequence is located within the target nucleic acid sequence between the portions of the target nucleic acid sequence complementary to the first and second primers. In some embodiments the target cleavage sequence is located within or overlaps with portions of the target nucleic acid sequence complementary to the first and second primers. In some embodiments of all aspects, the complete extension of the primers is inhibited when the nucleic acid amplification products are cleaved with the agent, therefore preventing exponential amplification of the target nucleic acid sequence. In some embodiments of all aspects the exponential amplification of the target nucleic acid sequence indicates the presence of the polymorphism. In some embodiments of all aspects, the reduced or linear amplification of the target nucleic acid sequence indicates the absence of the polymorphism. In some embodiments of all aspects the target nucleic acid sequence is different from the target cleavage sequence of the agent and is amplified at a higher rate than a target nucleic acid sequence that comprises the target cleavage sequence.

In some embodiments of any of the aspects described here, the target nucleic acid is double-stranded or single-stranded nucleic acid molecules, such as DNA (e.g., cDNA, gDNA, mtDNA, etc.) or RNA (e.g., vRNA, mRNA, snRNA, rRNA, tRNA, etc.). In some embodiments of all aspects, the specific cleavage sequence is located within the target nucleic acid sequence between the portions of the target nucleic acid sequence complementary to the first and second primers. In some embodiments the target cleavage sequence is located within or overlaps with portions of the target nucleic acid sequence complementary to the first and second primers.

In some embodiments of any of the aspects described here, the agent is a restriction enzyme or endonuclease, a zinc finger, a CRISPR-nuclease, or a TALEN.

In some embodiments of any of the aspects described here, a sequence capable of being cleaved by the agent is absent from the target nucleic acid sequence, but is introduced in the amplification product following a first round of amplification by the nucleic acid amplification reaction. In some embodiments, the rate of amplification of the target nucleic acid sequence is inhibited following introduction of a cleavage site following the first round of amplification. In some embodiments of all aspects, the exponential amplification of the target nucleic acid sequence indicates the absence of a target nucleic acid sequence that can be cleaved by the agent. In some embodiments of all aspects, reduced or linear amplification of the target nucleic acid sequence indicates the presence of a target nucleic acid sequence that can be cleaved by the agent.

In some embodiments of any of the aspects described here, the agent is a restriction endonuclease that is Dde1 or Hpy166II. In some embodiments of all aspects, the agent is a nuclease. In some embodiments of all aspects, the nuclease is a restriction endonuclease that is naturally occurring. In some embodiments of all aspects, the agent is a restriction endonuclease that is artificial. In some embodiments of all aspects the artificial restriction endonuclease is generated by fusing a TAL effector DNA binding domain to a DNA cleavage domain. In some embodiments of all aspects, the artificial restriction endonuclease is generated by fusing a zinc finger DNA binding domain to a DNA cleavage domain. In some embodiments of all aspects, the nuclease is a CRISPR associated (Cas) nuclease. In some embodiments of all aspects, the Cas nuclease is Cas9. In some embodiments of all aspects, the nuclease is a CRISPR associated Cpf1 nuclease.

In some embodiments of any of the aspects described here, the mixture can include a probe labeled with a detectable label. In some embodiments of all aspects, the probe comprises an oligonucleotide complimentary to a portion of the target nucleic acid sequence at a position that is in between the portions of the target nucleic acid sequence that are complementary to the first and the second primers. In some embodiments the target cleavage sequence is located within or overlaps with portions of the target nucleic acid sequence complementary to the first and second primers. In some embodiments of all aspects, the detectable label is an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, or members of binding pairs. In some embodiments of all aspects, the label is a fluorescent marker, e.g., fluorescein, FAM, TAMRA (tetramethylrhodamine) or Texas Red^{™}.

In some embodiments of any of the aspects described here, the target nucleic acid sequence is a wild-type sequence or a variant sequence, wherein the wild-type sequence comprises the target cleavage sequence and the variant sequence comprises one or more single nucleotide polymorphism(s) (SNP) compared to the wild-type sequence and does not comprise the specific cleavage sequence. In some embodiments of all aspects, the target nucleic acid sequence is a wild-type sequence or a variant sequence, wherein the variant sequence comprises one or more single nucleotide polymorphism(s) (SNP) compared to a wild-type sequence and comprises the target cleavage sequence, and the wild-type sequence does not comprise the specific cleavage sequence.

In some embodiments of any of the aspects described here, the SNP is associated with a particular disease status or diagnosis. In some embodiments of all aspects, the SNP is associated with a diagnosis of sickle cell anemia. In some embodiments of all aspects, the SNP is associated with a diagnosis of a tumor or cancer. In some embodiments of all aspects, the SNP is associated with drug resistance or susceptibility.

In some embodiments of any of the aspects described here, the target cleavage sequence differs from the target nucleic acid sequence at one or more positions, wherein the sequence of the first primer is complementary to the target nucleic acid sequence, and wherein the second primer differs from the target nucleic acid sequence at one or more positions and comprises at least part of the target cleavage sequence. In some embodiments of all aspects, the second primer introduces the agent specific cleavage sequence when amplified with the variant sequence.

In another aspect, this disclosure features methods of determining the genotype of a target allele in a sample of double-stranded nucleic acids, that include: (a) contacting the target allele in a first reaction with a first primer, a second primer, a recombinase, a polymerase, a first agent capable of cleaving double-stranded nucleic acid at a first specific cleavage sequence; (b) performing amplification by extending the primers along the sequence of the target allele to produce amplified replication products of the target allele; (c) cleaving the target sequence and the amplified products with the first agent; (d) repeating amplification such that the nucleic acid molecules that comprise a sequence that is different from the first specific cleavage sequence at one or more positions are amplified at a higher rate than amplified products that comprise the first specific cleavage sequence; and (e) detecting the amplified products; and optionally, (f) contacting the target allele in a second reaction with a third primer, a fourth primer, a recombinase, a polymerase, a second agent capable of cleaving double-stranded nucleic acid at a second specific cleavage sequence, wherein the second specific cleavage sequence is different from the target allele sequence at one or more positions; wherein the sequence of the third primer is complementary to that of the target allele sequence, wherein the sequence of the fourth primer differs from the sequence of the target allele sequence at one or more positions and comprises at least part of the second specific cleavage sequence; (g) performing amplification as in (b) to produce a first and a second amplified replication product, wherein the first amplified replication products are an extension of the third primer and comprise an identical replication of the target nucleic acid, and the second amplified replication products are an extension of the fourth primer and comprise the sequence of the fourth primer: (h) cleaving the first and second amplified replication products of (g) with the second agent; (i) repeating amplification such that the amplified replication products with a sequence that is different from the second specific cleavage sequence at one or more positions are amplified at a higher rate than the amplified replication products with the second specific cleavage sequence; (j) detecting the amplified products; and (k) comparing the detection of (e) to the detection of (j).

In some embodiments of any of the aspects described here, the target allele comprises a wild-type sequence or a variant sequence that comprises one or more single nucleotide polymorphism(s) (SNPs) compared to the wild-type sequence. In some embodiments of all aspects, the second specific cleavage sequence differs from the wild-type sequence at two or more positions (SNPs), wherein the variant sequence comprises a first SNP of the two or more SNPs and the fourth primer comprises a second SNP of the two or more SNPs, such that the fourth primer introduces the agent specific cleavage sequence when amplified with the variant sequence. In some embodiments of all aspects the second amplified replication product comprises the second specific cleavage sequence. In some embodiments of all aspects, the amplified products are detected as in (e) and (j) at a multitude of times.

In some embodiments of any of the aspects described here, the target allele is also contacted with a probe labeled with a detectable label. In some embodiments of all aspects, the probe comprises an oligonucleotide complimentary to a portion of the target nucleic acid sequence at a position that is in between the portions of the target nucleic acid sequence that are complementary to the first and the second primers. In some embodiments the target cleavage sequence is located within or overlaps with portions of the target nucleic acid sequence complementary to the first and second primers. In some embodiments of all aspects, the detectable label is an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, or members of binding pairs. In some embodiments of all aspects, the label is a fluorescent marker that is fluorescein, FAM, TAMRA (tetramethylrhodamine), Texas Red^{™}, or any combination thereof.

In another aspect, this disclosure features methods of determining the state of a target nucleic acid including the steps of: a) combining a target nucleic acid having a target sequence that exists in a first state or a second state with reagents suitable to amplify the target sequence and a nuclease; b) performing amplification; and c) detecting the amplified target nucleic acid, wherein the target nucleic acid is amplified and detected if the target sequence exists in the first state but not if the target sequence exists in the second state. In some embodiments, step a) comprises combining the target nucleic acid with reagents suitable for isothermal amplification of the target sequence and the step b) comprises performing isothermal amplification. In some embodiments, the step a) comprises combining the target nucleic acid with RPA reagents and the step b) comprises performing RPA.

In some embodiments of any of the aspects described here, the target sequence in the first state and the second state differ by at least one nucleotide. In some embodiments of all aspects, the target sequence in the first state and the second state differ by one nucleotide. In some embodiments of all aspects, the second state comprises a wild-type target sequence and the first state comprises a variant target sequence comprising a single nucleotide mutation (SNP) compared to the wild-type target sequence. In some embodiments of all aspects, the first state comprises a wild-type target sequence and the second state comprises a variant target sequence comprising a SNP compared to the wild-type target sequence.

In some embodiments of any of the aspects described here, the SNP is comprised within a targeted cleavage site susceptible to cleavage by the first enzyme. In some embodiments of all aspects, the SNP is associated with a particular disease status or diagnosis. In some embodiments of all aspects, the SNP is associated with a diagnosis of sickle cell anemia. In some embodiments of all aspects, the SNP is associated with a diagnosis of a tumor or cancer. In some embodiments of all aspects, the SNP is associated with drug resistance or susceptibility.

In some embodiments of any of the aspects described here, the target allele is also combined with a probe labeled with a detectable label. In some embodiments of all aspects, the mixture also includes a probe labeled with a detectable label. In some embodiments of all aspects, the probe comprises an oligonucleotide complimentary to a portion of the target nucleic acid sequence at a position that is in between the portions of the target nucleic acid sequence that are complementary to the first and the second primers. In some embodiments the target cleavage sequence is located within or overlaps with portions of the target nucleic acid sequence complementary to the first and second primers. In some embodiments of all aspects, the detectable label is selected from a group consisting of an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, and members of binding pairs. In some embodiments of all aspects, the label is a fluorescent marker that is selected from the group consisting of fluorescein, FAM, TAMRA (tetramethylrhodamine) and Texas Red^{™}.

In some embodiments of any of the aspects described here, the nuclease is a naturally occurring restriction endonuclease. In some embodiments of all aspects, the nuclease is an artificial restriction endonuclease. In some embodiments of all aspects, the artificial restriction endonuclease is generated by fusing a TAL effector DNA binding domain to a DNA cleavage domain. In some embodiments of all aspects, the artificial restriction endonuclease is generated by fusing a zinc finger DNA binding domain to a DNA cleavage domain. In some embodiments of any of the aspects described here, the nuclease is a CRISPR associated (Cas) nuclease. In some embodiments of all aspects, the Cas nuclease is Cas9. In some embodiments of any of the aspects described here, the nuclease is a CRISPR associated (Cpf1) nuclease.

In some embodiments of any of the aspects described here, the target nucleic acid is genomic DNA. In some embodiments of all aspects, the target nucleic acid is a double-stranded DNA molecule. In some embodiments of all aspects, the target nucleic acid is comprised in a gene from a subject. In some embodiments of all aspects, the target nucleic acid is comprised in each of a pair of genes from a subject.

In some embodiments of any of the aspects described here, the method of determining the state of a target nucleic acid further includes the steps of: d) combining the target nucleic acid having a target sequence that exists in a first state and a second state with reagents suitable to amplify the target sequence and a second enzyme; e) performing amplification; and f) detecting the amplified target nucleic acid, wherein the target nucleic acid is amplified and detected if the target sequence exists in the second state but not if the target sequence exists in the first state. In some embodiments of all aspects, the target nucleic acid is further combined with a probe labeled with a detectable label. In some embodiments, the step d) comprises combining the target nucleic acid with reagents suitable for isothermal amplification of the target sequence and the step e) comprises performing isothermal amplification. In some embodiments, the step d) comprises combining the target nucleic acid with RPA reagents and the step d) comprises performing RPA.

In some embodiments of any of the aspects described here, the probe comprises an oligonucleotide complimentary to a portion of the target nucleic acid sequence at a position that is in between the portions of the target nucleic acid sequence that are complementary to the first and the second primers. In some embodiments the target cleavage sequence is located within or overlaps with portions of the target nucleic acid sequence complementary to the first and second primers. In some embodiments of all aspects, the detectable label is an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, or members of binding pairs. In some embodiments of all aspects, the label is a fluorescent marker that is selected from the group consisting of fluorescein, FAM, TAMRA (tetramethylrhodamine) or Texas Red^{™}.

In some embodiments of any of the aspects described here, the second state comprises a wild-type target sequence and the first state comprises a variant target sequence comprising a SNP compared to the wild-type target sequence. In some embodiments of all aspects, the first state comprises a wild-type target sequence and the second state comprises a variant target sequence comprising a SNP compared to the wild-type target sequence. In some embodiments of all aspects, the SNP is comprised within a targeted cleavage site susceptible to cleavage by both the first enzyme and the second enzyme.
the target nucleic acid being present in each of a pair of genes from the subject and corresponding with either the wild-type allele or a variant allele of the gene;

In another aspect, this disclosure features methods of genotyping the DNA of a subject, including the steps: a) combining a target nucleic acid having a target sequence with reagents suitable to amplify the target sequence and either a first enzyme or a second enzyme, the target nucleic acid being present in each of a pair of genes from the subject and corresponding with either the wild-type allele or a variant allele of the gene; b) performing amplification; and c) detecting the amplified target nucleic acid, wherein: i) in the presence of the first enzyme, the target nucleic acid is amplified and detected if the target sequence corresponds to the wild-type allele but not if the target sequence corresponds to the variant allele, and ii) in the presence of the second enzyme, the target nucleic acid is amplified and detected if the target sequence corresponds to the variant allele but not if the target sequence corresponds to the wild-type allele. In some embodiments, the step a) comprises combining the target nucleic acid with reagents suitable for isothermal amplification of the target sequence and the step b) comprises performing isothermal amplification. In some embodiments, the step a) comprises combining the target nucleic acid with RPA reagents and the step b) comprises performing RPA.

In some embodiments of any of the aspects described here, the target sequence corresponding to the variant allele comprises a SNP compared to the target sequence corresponding to the wild-type allele. In some embodiments of all aspects, the SNP is comprised within a targeted cleavage site susceptible to cleavage by both the first enzyme and the second enzyme. In some embodiments of all aspects, the SNP is associated with a particular disease status or diagnosis. In some embodiments of all aspects, the SNP is associated with a diagnosis of sickle cell anemia. In some embodiments of all aspects, the SNP is associated with a diagnosis of a tumor or cancer. In some embodiments of all aspects, the SNP is associated with drug resistance or susceptibility.

In some embodiments of any of the aspects described here, the first enzyme and the second enzyme are each a nuclease. In some embodiments of all aspects, the nuclease is naturally occurring restriction endonuclease. In some embodiments of all aspects, the nuclease is an artificial restriction endonuclease. In some embodiments of all aspects, the artificial restriction endonuclease is generated by fusing a TAL effector DNA binding domain to a DNA cleavage domain. In some embodiments of all aspects, the artificial restriction enzyme is generated by fusing a zinc finger DNA binding domain to a DNA cleavage domain. In some embodiments of all aspects, the nuclease is a CRISPR associated (Cas) nuclease. In some embodiments of all aspects, the Cas nuclease is Cas9. In some embodiments of all aspects, the nuclease is a CRISPR associated (Cpf1) nuclease.

In some embodiments of any of the aspects described here, the target nucleic acid of a) is further combined with a probe labeled with a detectable label. In some embodiments of all aspects, the probe comprises an oligonucleotide complimentary to a portion of the target nucleic acid sequence at a position that is in between the portions of the target nucleic acid sequence that is complementary to the first and the second primers. In some embodiments the target cleavage sequence is located within or overlaps with portions of the target nucleic acid sequence complementary to the first and second primers. In some embodiments of all aspects, the detectable label is selected from a group consisting of an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, and members of binding pairs. In some embodiments of all aspects, the label is a fluorescent marker that is selected from the group consisting of fluorescein, FAM, TAMRA (tetramethylrhodamine) and Texas Red^{™}.

In another aspect, this disclosure features a method of determining the state of a target nucleic acid comprising the steps of: a) providing a sample comprising a target nucleic acid having a target sequence that exists in a first state or a second state; b) cleaving the target sequence if it exists in the first state but not the second state; c) performing amplification of the target sequence, if it has not been cleaved in b); and d) detecting amplified target nucleic acid, wherein the detection of amplified target nucleic acid indicates the target sequence exists in the second state and the absence of detected amplified target nucleic acid indicates that the target sequence exits in the first state. In some embodiments of all aspects, performing amplification comprises performing isothermal amplification. In some embodiments of all aspects, performing amplification comprises performing RPA.

In some embodiments of all aspects, the target sequence in the first state and the second state differ by at least one nucleotide. In some embodiments of all aspects, the target sequence in the first state and the second state differ by one nucleotide. In some embodiments of all aspects, the second state comprises a wild-type target sequence and the first state comprises a variant target sequence comprising a single nucleotide mutation (SNP) compared to the wild-type target sequence. In some embodiments of all aspects, the first state comprises a wild-type target sequence and the second state comprises a variant target sequence comprising a SNP compared to the wild-type target sequence. In some embodiments of all aspects, the SNP is comprised within a targeted cleavage site susceptible to cleavage by the first enzyme.

In some embodiments of all aspects, the SNP is associated with a particular disease status or diagnosis. In some embodiments of all aspects, the SNP is associated with a diagnosis of sickle cell anemia. In some embodiments of all aspects, the SNP is associated with a diagnosis of a tumor or cancer. In some embodiments of all aspects, the SNP is associated with drug resistance or susceptibility.

In some embodiments of all aspects, the target nucleic acid of (a) is further combined with a probe labeled with a detectable label. In some embodiments of all aspects, the sample of (a) further comprises a probe labeled with a detectable label. In some embodiments of all aspects, the probe comprises an oligonucleotide complimentary to a portion of the target nucleic acid sequence at a position that is in between the portions of the target nucleic acid sequence that are complementary to the first and the second primers, or located within or overlapping with portions of the target nucleic acid sequence that are complementary to the first and second primers. In some embodiments of all aspects, the detectable label is selected from a group consisting of an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, and members of binding pairs. In some embodiments of all aspects, the label comprises a fluorescent marker that is selected from the group consisting of fluorescein, FAM, TAMRA (tetramethylrhodamine) and Texas Red^{™}.

In some embodiments of all aspects, the target nucleic acid sequence is cleaved by a nuclease. In some embodiments of all aspects, the nuclease is a naturally occurring restriction endonuclease. In some embodiments of all aspects, the nuclease is an artificial restriction endonuclease. In some embodiments of all aspects, the artificial restriction endonuclease is generated by fusing a TAL effector DNA binding domain to a DNA cleavage domain. In some embodiments of all aspects, the artificial restriction endonuclease is generated by fusing a zinc finger DNA binding domain to a DNA cleavage domain. In some embodiments of all aspects, the nuclease is a CRISPR associated (Cas) nuclease. In some embodiments of all aspects, the Cas nuclease is Cas9.

In some embodiments of all aspects, the target nucleic acid is genomic DNA. In some embodiments of all aspects, the target nucleic acid is a double-stranded DNA molecule. In some embodiments of all aspects, the target nucleic acid is comprised in a gene from a subject. In some embodiments of all aspects, the target nucleic acid is comprised in each of a pair of genes from a subject.

The compositions disclosed herein comprise reagents suitable for NEAR amplification of a target sequence. In some embodiments of all aspects, the methods disclosed herein comprise combining a target nucleic acid with reagents suitable for NEAR amplification and performing NEAR amplification.

The term "one or more" or "at least one" as used in the present invention stands for 1, 2, 3, 4, 5, 6, 7, 8, 9, 10 compounds or even more.

The terms "first" and "second" are used in this disclosure in their relative sense only. It will be understood that, unless otherwise noted, those terms are used merely as a matter of convenience in the description of one or more of the embodiments. The terms "first" and "second" are only used to distinguish one element from another element, and the scope of the rights of the disclosed technology should not be limited by these terms. For example, a first element may be designated as a second element, and similarly the second element may be designated as the first element.

A "sample" as used herein refers to a biological material that is isolated from its environment (e.g., blood or tissue from an animal, cells, or conditioned media from tissue culture) and is suspected of containing, or known to contain an analyte or other desired material. A sample can also be a partially purified fraction of a tissue or bodily fluid, e.g., from a subject having a specific disease or condition. A reference sample can be a "normal" sample, from a donor not having the disease or condition. A reference sample can also be from an untreated donor or cell culture not treated with an active agent (e.g., no treatment or administration of vehicle only) or not subjected to conditions to induce a disease state. A reference sample can also be taken at a "zero time point" prior to contacting the cell with the agent to be tested.

The terms "increased", "increase" or "up-regulated" are all used herein to generally mean an increase by a statistically significant amount; for the avoidance of any doubt, the terms "increased" or "increase" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 0.5-fold, or at least about a 1.0-fold, or at least about a 1.2-fold, or at least about a 1.5-fold, or at least about a 2-fold, or at least about a 3-fold, or at least about a 4-fold, or at least about a 5-fold or at least about a 10-fold increase, or any increase between 1.0-fold and 10-fold or greater as compared to a reference level.

The terms "decrease", "decreased", "reduced", "reduction" or 'downregulated" are all used herein generally to mean a decrease by a statistically significant amount. However, for avoidance of doubt, "reduced", "reduction", "decreased" or "decrease" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (i.e. absent level as compared to a reference sample), or any decrease between 10-100% as compared to a reference level, or at least about a 0.5-fold, or at least about a 1.0-fold, or at least about a 1.2-fold, or at least about a 1.5-fold, or at least about a 2-fold, or at least about a 3-fold, or at least about a 4-fold, or at least about a 5-fold or at least about a 10-fold decrease, or any decrease between 1.0-fold and 10-fold or greater as compared to a reference level.

The articles "a" and "an" are used herein to refer to one or to more than one (i.e., to at least one) of the grammatical object of the article. By way of example, "an element" means one element or more than one element.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

The invention is defined in the appended claims.

### DESCRIPTION OF DRAWINGS

FIG 1 depicts an exemplary RPA reaction method according to the disclosure for the detection of the BCR-ABL polymorphism responsible for the T315I mutation. Sequence shown with select Ddel1 sites in grey (SEQ ID NO: 8).
FIG 2 is a graph of RPA detecting of BCR-ABL T315I mutation.
FIG 3 is a graph of RPA detecting trace amounts of the BCR-ABL T315I mutation.
FIG 4 depicts an exemplary RPA reaction method according to the disclosure for the detection of rs334 polymorphism. Sequence is shown with the mismatch in grey (SEQ ID NO: 9).
FIG 5 depicts an exemplary RPA reaction method according to the disclosure for the detection of rs334 polymorphism.
FIG 6. is a graph of a rs334 genotyping RPA assay.
FIG 7 is a graph comparing hypothetical genotyping RPA assays of a homozygous wild type, heterozygous and homozygous variant templates.
FIG 8 is a schematic demonstrating the genotyping system using CRISPR-RPA for the detection of the rs334 polymorphism. In the left panel, WT specific crRNA sequence (SEQ ID NO: 10) is shown aligned with rs334 wild type sequence (SEQ ID NO: 11)(top) and rs334 variant sequence (SEQ ID NO: 12)(bottom). In the right panel, variant specific crRNA sequence (var specific crRNA sequence)(SEQ ID NO: 13) is shown aligned with rs334 wild type sequence (SEQ ID NO: 14)(top) and rs334 variant sequence (SEQ ID NO: 15)(bottom).

### DETAILED DESCRIPTION

This disclosure is based in part on the discovery that it is possible to detect polymorphisms of a target sequence using RPA and trace amounts of target nucleic acid. To that end, the present application discloses a composition for detecting a polymorphism in a target nucleic acid sequence. Also to that end, the present application discloses methods for detecting a polymorphism in a target nucleic acid sequence.

Nucleic acids (e.g., polynucleotides) suitable for amplification in connection with the present methods include double-stranded and single-stranded nucleic acid molecules, such as DNA and RNA molecules. The polynucleotides may be of genomic, chromosomal, plasmid, mitochondrial, cellular, and viral nucleic acid origin. For double stranded polynucleotides, the amplification may be of either one or both strands.

As described here, RPA employs enzymes, known as recombinases, that are capable of pairing oligonucleotide primers with homologous sequences in template double-stranded nucleic acid. In this way, DNA synthesis is directed to defined points in a template double-stranded nucleic acid. Using two or more sequence-specific (e.g., gene-specific) primers, an exponential amplification reaction is initiated if the template nucleic acid is present. The reaction progresses rapidly and results in specific amplification of a sequence present within the template double-stranded nucleic acid from just a few copies of the template nucleic acid to detectable levels of the amplified products within minutes. RPA methods are disclosed, e.g., in US 7,270,981; US 7,399,590; US 7,666,598; US 7,435,561; US 2009/0029421; and WO 2010/141940.

Is some aspects, the present application discloses compositions and methods for detecting a polymorphism in a target nucleic acid sequence using Nicking and Extension Amplification Reaction (NEAR). NEAR methods are disclosed, e.g., in U.S. 2009/0081670 and U.S. 2009/0017453.

The composition disclosed herein can contain a set of primers that amplify the target nucleic acid sequence. The primers can comprise of sequences that are complementary to the target nucleic acid sequence or that differ from the target nucleic acid sequence at one or more positions. As described herein, the amplification product, of RPA with a primer that differs from the target nucleic acid sequence at one or more positions, can differ from the target sequence at the one or more positions. The amplification product of the RPA reaction described herein can comprise a target cleavage sequence.

The set of primers can amplify the target nucleic acid sequence or they can introduce a sequence that differs from the target nucleic acid sequence at one or more positions. This introduced sequence can consist of a target cleavage sequence. The first primer can be complementary to the target nucleic acid sequence. The second primer can comprise a first portion that is complementary to the target nucleic acid sequence and a second portion that is different from the target nucleic acid sequence at one or more positions. When the two primers amplify the nucleic acid sequence the second primer incorporates the one or more different positions into the amplified products. This amplified region is different from the target nucleic acid sequence at the one or more positions and can consist of the target cleavage sequence.

The RPA composition disclosed herein contains a recombinase, which may originate from prokaryotic, viral or eukaryotic origin. Exemplary recombinases include RecA and UvsX (e.g., a RecA protein or UvsX protein obtained from any species), and fragments or mutants thereof, and combinations thereof. The RecA and UvsX proteins can be obtained from any species. RecA and UvsX fragments or mutant proteins can also be produced using the available RecA and UvsS protein and nucleic acids sequences, and molecular biology techniques (see, e.g., the mutant forms of UvsX described in U.S. Patent No. 8,071,308). Exemplary UvsX proteins include those derived from myoviridae phages, such as T4, T2, T6, Rb69, Aeh1, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, and phage LZ2. Additional exemplary recombinase proteins include archaebacterial RADA and RADB proteins and eukaryotic (e.g., plant, mammal, and fungal) Rad51 proteins (e.g., RAD51, RAD51B, RAD51C, RAD51D, DMC1, XRCC2, XRCC3, and recA) (see, e.g., Lin et al., Proc. Natl. Acad. Sci. U.S.A. 103:10328-10333, 2006).

In any process of this disclosure, the recombinase (e.g., UvsX) may be a mutant or hybrid recombinase. In some embodiments, the mutant UvsX is an Rb69 UvsX that includes at least one mutation in the Rb69 UvsX amino acid sequence, wherein the mutation is selected from the group consisting of (a) an amino acid which is not histidine at position 64, a serine at position 64, the addition of one or more glutamic acid residues at the C-terminus, the addition of one or more aspartic acid residues at the C-terminus, and a combination thereof. In other embodiments, the mutant UvsX is a T6 UvsX having at least one mutation in the T6 UvsX amino acid sequence, wherein the mutation is selected from the group consisting of (a) an amino acid which is not histidine at position 66; (b) a serine at position 66; (c) the addition of one or more glutamic acid residues at the C-terminus; (d) the addition of one or more aspartic acid residues at the C-terminus; and (e) a combination thereof. Where a hybrid recombinase protein is used, the hybrid protein may, for example, be a UvsX protein that includes at least one region that includes an amino acid sequence derived from a different UvsX species. The region may be, for example, the DNA-binding loop-2 region of UvsX.

The DNA polymerase disclosed herein may be a eukaryotic or prokaryotic polymerase. Examples of eukaryotic polymerases include pol-alpha, pol-beta, pol-delta, pol-epsilon, and mutants or fragments thereof, or combinations thereof. Examples of prokaryotic polymerase include *E*. *coli* DNA polymerase I (e.g., Klenow fragment), bacteriophage T4 gp43 DNA polymerase, Bacillus stearothermophilus polymerase I large fragment, Phi-29 DNA polymerase, T7 DNA polymerase, Bacillus subtilis Pol I, Staphylococcus aureus Pol I, *E. coli* DNA polymerase I, *E. coli* DNA polymerase II, *E. coli* DNA polymerase III, *E. coli* DNA polymerase IV, *E*. *coli* DNA polymerase V, and mutants or fragments thereof, or combinations thereof. In some embodiments, the DNA polymerase lacks 3'-5' exonuclease activity. In some embodiments, the DNA polymerase has strand-displacing properties, e.g., large fragments of prokaryotic polymerases of class pol I or pol V.

In some embodiments, one or more probes (e.g., molecular beacon probes) are labeled with one or more detectable labels. Exemplary detectable labels include haptens, enzymes, enzyme substrates, coenzymes, enzyme inhibitors, fluorophores, quenchers, chromophores, magnetic particles or beads, redox sensitive moieties (e.g., electrochemically active moieties), luminescent markers, radioisotopes (including radionucleotides), and members of binding pairs. More specific examples include fluorescein, phycobiliprotein, tetraethyl rhodamine, and beta-galactosidase. Binding pairs may include biotin/streptavidin, biotin/avidin, biotin/neutravidin, biotin/captavidin, epitope/antibody, protein A/immunoglobulin, protein G/immunoglobulin, protein L/immunoglobulin, GST/glutathione, His-tag/Metal (e.g., nickel, cobalt or copper), antigen/antibody, FLAG/M1 antibody, maltose binding protein/maltose, calmodulin binding protein/calmodulin, enzyme-enzyme substrate, receptor-ligand binding pairs, and analogs and mutants of the binding pairs.

As used herein, the terms "fluorescence label" and "fluorophore" are used interchangeably and refer to any substance that emits electromagnetic energy at a certain wavelength (emission wavelength) when the substance is illuminated by radiation of a different wavelength (excitation wavelength) and is intended to encompass a chemical or biochemical molecule or fragments thereof that is capable of interacting or reacting specifically with an analyte of interest in a sample to provide one or more optical signals.

Representative fluorophores for use in the methods provided herein include, for example, FAM, (tetramethylrhodamine) Texas Red^{™}, green fluorescent protein, blue fluorescent protein, red fluorescent protein, fluorescein, fluorescein 5-isothiocyanate (FITC), cyanine dyes (Cy3, Cy3.5, Cy5, Cy5.5, Cy7), Bodipy dyes (Invitrogen) and/or Alexa Fluor dyes (Invitrogen), dansyl, Dansyl Chloride (DNS-C1), 5-(iodoacetamida)fluorescein (5-IAF, 6- acryloyl-2-dimethylaminonaphthalene (acrylodan), 7-nitrobenzo-2-oxa-1,3,-diazol-4-yl chloride (NBD-Cl), ethidium bromide, Lucifer Yellow, rhodamine dyes (5-carboxyrhodamine 6G hydrochloride, Lissamine rhodamine B sulfonyl chloride, rhodamine-B-isothiocyanate (RITC (rhodamine-B-isothiocyanate), rhodamine 800); tetramethylrhodamine 5 -(and 6-)isothiocyanate (TRITC)), Texas Red^{™}, sulfonyl chloride, naphthalamine sulfonic acids including but not limited to 1- anilinonaphthalene-8 -sulfonic acid (ANS) and 6-(p-toluidinyl)naphthalen-e-2-sulfonic acid (TNS), Anthroyl fatty acid, DPH, Parinaric acid, TMA-DPH, Fluorenyl fatty acid, Fluorescein-phosphatidylethanolamine, Texas red-phosphatidylethanolamine, Pyrenyl- phophatidylcholine, Fluorenyl-phosphotidylcholine, Merocyanine 540, Naphtyl Styryl, 3,3'dipropylthiadicarbocyanine (diS-C3-(5)), 4-(p-dipentyl aminostyryl)-l-methylpyridinium (di-5-ASP), Cy-3 lodo Acetamide, Cy-5-N- Hydroxysuccinimide, Cy-7-Isothiocyanate, IR-125, Thiazole Orange, Azure B, Nile Blue, Al Phthalocyanine, Oxaxine 1, 4', 6-diamidino-2-phenylindole. (DAPI), Hoechst 33342, TOTO, Acridine Orange, Ethidium Homodimer, N(ethoxycarbonylmethyl)-6-methoxyquinolinium (MQAE), Fura-2, Calcium Green, Carboxy SNARF-6, BAPTA, coumarin, phytofluors, Coronene, and metal-ligand complexes.

It should be noted that a fluorescence quencher is also considered a detectable label. For example, the fluorescence quencher may be contacted to a fluorescent dye and the amount of quenching is detected.

Haptens for use in the methods provided herein include, for example, digoxigenin, glutathione and biotin.

Enzymes for use in the methods provided herein include, for example, alkaline phosphatase (AP), beta-galactosidase, horse radish peroxidase (HRP), soy bean peroxidase (SBP), urease, beta-lactamase and glucose oxidase.

The embodiments described herein can also include an agent capable of cleaving a particular target nucleic acid sequence or a nuclease. As used herein, the term "nuclease" refers to enzymes capable of catalyzing the hydrolysis of nucleic acids, cleaving the phosphodiester bonds between the nucleotide subunits of nucleic acids. A "restriction nuclease" is a nuclease that targets and cleaves a nucleic acid molecule at or near specific recognition nucleotide sequences known as restriction sites. Nucleases may be further divided into endonucleases (i.e., enzymes that cleave the phosphodiester bond within a polynucleotide chain) and exonucleases (i.e., enzymes that work by cleaving nucleotides one at a time from the end (exo) of a polynucleotide chain), although some of the enzymes may fall in both categories. The nuclease can be a naturally occurring restriction endonuclease or an artificial endonuclease.

Throughout the disclosure, the terms "restriction enzyme" and "restriction endonuclease" are used interchangeably and refer to a nuclease that targets and cleaves a double stranded nucleic acid at or near a restriction site, cutting of both strands of the target nucleic acid to yield a blunt ended or sticky ended cut site. Different restriction endonucleases recognize different recognition sequences and are known to persons skilled in the art and are available from various commercial sources.

In some embodiments of all aspects, the agent capable of cleaving double stranded nucleic acid at a target cleavage sequence is a "restriction endonuclease." Through the use of the restriction endonuclease, a specific nucleotide sequence is targeted as determined by the specific restriction site, resulting in cleavage of both strands to yield a blunt ended or sticky ended fragment. The resulting fragment having a blunt or sticky end prevents further replication of both strands between the regions to nucleic acid that are complementary to the forward and reverse primers. As a result there is no replication of the complimentary strand and thus no exponential increase in the number of copies of the nucleic acid to which the probe will bind.

In some embodiments of all aspects, the agent capable of cleaving double stranded nucleic acid at a target cleavage sequence is not a DNA glycosylase or a glycosylase/abasic (AP) lyases. DNA glycosylase or a glycosylase/abasic (AP) lyases are a family of enzymes involved in base excision repair, by which damaged bases in DNA are removed and replaced. Thus, the agent described herein is not, for example, any one of ExoIII, Fpg, Nfo, Nth, MutY, MutS, MutM, *E. coli* MUG, human MUG, human Ogg1, a vertebrate Nei-like (Neil) glycosylase, uracil glycosylase, or hypoxanthine-DNA.

Examples of the restriction endonucleases described herein include AatII, AbaSI, Acc65I, AccI, Acil, Acll, Acul, AfeI, AfIII, AfIIII, Agel, Ahdl, AleI, AluI, AlwI, AlwNI, ApaI, ApeKI, ApoI, BamHI, BanI, BbvI, BccI, BcgI, BgII, BgII, BmgBI, BmrI, BpmI, BsaAI, BsaBI, BsaHI, BsaI, BsgI, BsII, BsmAI, CspCI, ClaI, Cac8I, DdeI, DpnI, DrdI, EaeI, EagI, EarI, EcoRI, FatI, FseI, HaeII, HhaI, HindIII, I-CeuI, KasI, KpnI, LpnPI, MboI, MboII, MfeI, MluCl, Mlyl, MmeI, MseI, Msll, MspI, NaeI, NarI, NdeI, HheI, NlaIII, NotI, PacI, PaeR7I, PciI, PhoI, PleI, PmeI, PshAI, PspGI, PstI, PvuI, RsaI, SacI, SacII, SaII, SapI, SbfI, ScaI, SexAI, SfaNI, SfoI, SmII, SpeI, StuI, StyD4I, Tfil, TseI, Tsp451, Tth111I, XbaI, Xcml, XhoI, Xmal, Zral, and any functional analogs or homologs thereof. One of skill in the art would appreciate that any restriction endonuclease could be used, including for example, an artificial restriction enzyme (i.e., an artificial nuclease). Artificial restriction endonucleases may include a TAL effector DNA binding domain fused to a DNA cleavage domain or a zinc finger domain fused to a DNA cleavage domain. The nuclease can be Clustered Regularly Interspaced Short Palindromic Repeats (CRISPR) and components thereof, e.g., CRISPR associated nuclease. In some embodiments the CRISPR associated nuclease is a Cas nuclease, e.g., Cas9. In some embodiments the CRISPR associated nuclease is Cpf1. The examples listed here are non-limiting and the embodiments disclosed herein can include a variety of agents capable of cleaving a particular sequence, e.g., a sequence-specific nuclease.

In some embodiments, the agent capable of cleaving a particular target nucleic acid sequence or a nuclease is not ExoIII, Fpg, Nfo, Nth, MutY, MutS, MutM, *E. coli* MUG, human MUG, human Ogg1, a vertebrate Nei-like (Neil) glycosylase, uracil glycosylase, hypoxanthine-DNA.

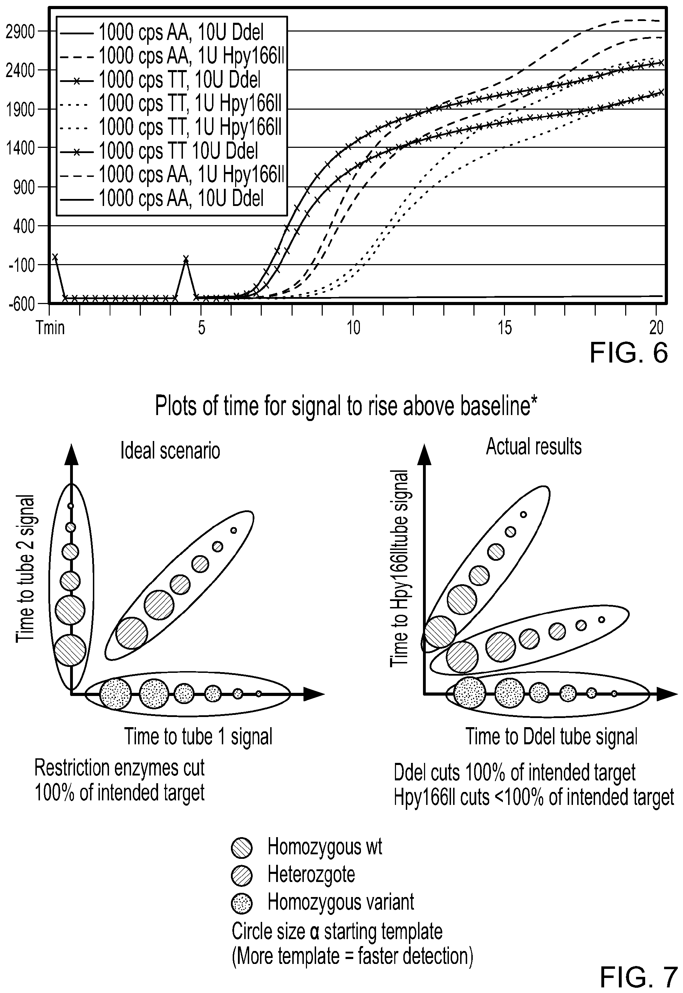

The CRISPR-nuclease system, including, for example the CRISPR-Cas system and the CRISPR-Cpf1 system, is an example of sequence-specific nuclease that can be used in the embodiments described herein. The CRISPR system works by recruiting the nuclease, e.g., Cas or Cpf1, to a specific DNA target using a short RNA molecule. These short RNA molecules recognize specific DNA targets that can then be cleaved by a nuclease, e.g., Cas or Cpf1. Some systems include at least an endonuclease (e.g., Cas9), CRISPR RNA (crRNA) and tracer RNA (tracrRNA) (see FIG. 7). The short RNA described herein can guide the CRISPR-nuclease system to the target nucleic acid sequence.

Additionally, a transcription activator-like effector Nuclease (TALEN) can be used as the nuclease. TALENs are made by fusing a TAL effector DNA-binding domain to a DNA cleavage domain. The TALENs can be designed to bind and cleave a target nucleic acid sequence.

Additionally, an artificial nuclease used in the embodiments described herein can be a zinc finger nuclease. A zinc finger nuclease is a fusion of a zinc finger binding domain and a DNA-cleavage domain. The zinc finger binding domain can bind to DNA, RNA and/or protein in a sequence-specific manner. A zinc finger nuclease can be used for the specific targeting and cleavage of a target nucleic acid sequence.

Additionally, one or more single-stranded DNA binding proteins can be used to stabilize nucleic acids during the various exchange reactions that are ongoing in the reaction. The one or more single-stranded DNA binding proteins can be derived or obtained from any species, e.g., from a prokaryotic, viral or eukaryotic species. Non-limiting exemplary single-stranded DNA binding proteins include *E. coli* SSB and those derived from myoviridae phages, such as T4, T2, T6, Rb69, Aeh1, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, and phage LZ2. Additional examples of single-stranded DNA binding proteins include A. denitrificans Alide _2047, Burkholderia thailandensis BthaB_33951, Prevotella pallens HMPREF9144_0124, and eukaryotic single-stranded DNA binding protein replication protein A.

Any of the processes of this disclosure may be performed in the presence of a crowding agent. In some embodiments, the crowding agent may include one or more of polyethylene glycol, polyethylene oxide, polyvinyl alcohol, polystyrene, Ficoll, dextran, poly(vinylpyrrolidone) (PVP), and albumin. In some embodiments, the crowding agent has a molecular weight of less than 200,000 daltons. Further, the crowding agent may be present, e.g., in an amount of about 0.5% to about 15% weight to volume (w/v).

If a recombinase loading protein is used, the recombinase loading protein may be of prokaryotic, viral or eukaryotic origin. Exemplary recombinase loading proteins include *E. coli* RecO, *E. coli* RecR, UvsY, and mutants or fragments thereof, or combinations thereof. Exemplary UvsY proteins include those derived from myoviridae phages, such as T4, T2, T6, Rb69, Aeh1, KVP40, *Acinetobacter* phage *133, Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, and phage LZ2. In any of the processes of this disclosure, the recombinase loading agent may be derived from a myoviridae phage. The myoviridae phage may be, for example, T4, T2, T6, Rb69, Aeh1, KVP40, *Acinetobacter* phage 133, *Aeromonas* phage 65, cyanophage P-SSM2, cyanophage PSSM4, cyanophage S-PM2, Rb14, Rb32, *Aeromonas* phage 25, *Vibrio* phage nt-1, phi-1, Rb16, Rb43, Phage 31, phage 44RR2.8t, Rb49, phage Rb3, or phage LZ2.

Further, any of the processes of this disclosure may be performed with a blocked primer. A blocked primer is a primer which does not allow elongation with a polymerase. Where a blocked primer is used, an unblocking agent can be used to unblock the primer to allow elongation. The unblocking agent may be an endonuclease or exonuclease which can cleave the blocking group from the primer. Exemplary unblocking agents include *E. coli* exonuclease III and *E. coli* endonuclease IV.

The processes of this disclosure include the detection of a target nucleic acid sequence where the target nucleic acid may include a natural cut site for a restriction endonuclease or a nuclease. Additionally a cut site may be introduced into the target nucleic acid sequence by the amplification of the target sequence with primers that differ from the target nucleic acid sequence at one or more positions. The introduction of an artificial cut site or a cut site that was not found in the target nucleic acid sequence can be used to detect the target nucleic acid sequence or the presence of a SNP in the target nucleic acid sequence.

The processes described herein can also be performed in parallel using a variety of the restriction endonuclease or nucleases described herein. The detection of the amplification products can be performed in parallel and the rates of amplification compared to a reference sample. The processes described herein can be used for the detection of a target sequence, or the genotyping of a sequence.

In some of the embodiments, monitoring the rate of increase of nucleic acid amplification products can include determining the number or proportion of amplification products in the reaction mixture over time. In some embodiments the amplification products are detected using fluorescence, phase contrast microscopy, luminescent detection, spectral (color) detection, magnetic detection, radioisotopic detection and/or electrochemical detection. One of skill in the art would appreciate that any technique known in the art to measure the amount of nucleic acid amplification products in a mixture can be used to detect amplification products and monitor the increase in amplification products over time. In some of the RPA processes described herein a detectable label may be used to monitor the progress (the production of amplification products) of the RPA reaction.

In some embodiments, if the primers are labeled, monitoring may involve detecting a label in an amplimer. Since amplimers would be expected to be larger than the primers used, detection may involve, for example gel electrophoresis and the detection of the proper sized amplimer. Alternatively, labeled amplimers may be separated by labeled primers using a more rapid process such as column chromatography (including spin columns, push columns and the like). Since the RPA methods of the invention have high specificity and low artifact production (high signal to noise), monitoring may involve performing RP A using nucleotides attached to detectable labels and measuring the amount of labels attached to high molecular weight nucleic acid (e.g., nucleic acid of more than 100 bases in length). For example, radioactive dNTPs may be used and the progress of the RP A reaction may be monitored by following the incorporation of radiation into high molecular weight DNA. Techniques that monitor incorporation of nucleotides into high molecular weight DNA include gel electrophoresis, size exclusion chromatography (e.g., using conventional, spin and push columns) and acid or alkali precipitation.

In a further aspect, the present disclosure provides compositions and methods for detecting for the presence or absence of the polymorphism of the ABL gene coding for the ABL T315I mutation, the method comprising: (1) mixing a first and second primer for amplifying the ABL gene, a recombinase, a polymerase, DdeI (or a restriction endonuclease or nuclease that recognizes the wild type ABL gene sequence) and nucleic acid comprising the ABL gene of a patient for which the presence or absence of the polymorphism in the ABL gene is to be determined; (2) performing a nucleic acid amplification reaction such as RPA of the mixture to amplify the ABL gene; and (3) monitoring the rate of increase in the amplification products, wherein the exponential rate of increase of nucleic acid amplification is indicative of the presence of a polymorphism in the ABL gene. The wild type ABL gene sequence would be cleaved by the restriction endonuclease or nuclease specific for that sequence (i.e. DdeI) and thus, exponential amplification would not occur. However in the presence of a polymorphism ABL T315I mutation, the restriction endonuclease DdeI would not recognize the sequence and therefore would not cleave the sequence, leading to exponential amplification.

It will be appreciated that in this aspect, the ABL T315I mutation may be present on a fusion protein, particularly a BCR-ABL fusion protein. In such a case, the 315* amino acid in the fusion protein may not be the same position as the 315* amino acid in non-fused ABL. However, a skilled person would readily be able to identify the corresponding amino acid position in the fusion protein, since a person skilled in the art can readily align similar sequences and locate the same mutant positions.

In a further aspect, the present disclosure provides a compositions and methods of detecting for the presence of the rs334 polymorphism in human beta-globin gene (i.e. variant-T comprising the sequence GTGGAG), the method comprising: two reactions wherein the reactions both comprise (a) mixing a first and second primer for amplifying the human beta-globin gene, a recombinase, a polymerase, a restriction endonuclease or nuclease and nucleic acid comprising the human beta-globin gene of a patient for which the presence or absence of the polymorphism in the human beta-globin gene is to be determined; (b) performing a nucleic acid amplification reaction such as RPA on the mixture to amplify the human beta-globin gene; and (c) monitoring the rate of increase in the amplification products, wherein the reverse primer includes a mismatch that upon replication introduces the sequence GNGGAC to the human beta-globin gene, wherein the restriction endonuclease or nuclease in the first of the two reactions is DdeI and the restriction endonuclease or nuclease in the second of the two reactions is Hpy166II, wherein the exponential rate of increase of nucleic acid amplification products in the first reaction is indicative of the presence of the rs334 polymorphism in the human beta-globin gene, and wherein the exponential rate of increase of nucleic acid amplification products in the second reaction is indicative of the wild type sequence of the human beta-globin.

It will be appreciated that in this aspect, the mutation may be present in a different position or gene, and the primers may introduce an alternative sequence mutation. However, a skilled person would readily be able to identify the introduced mutation in an amplified product and primers, since a person skilled in the art can readily align similar sequences and locate the mutant positions.

### Applications

The methods and compositions disclosed herein can be used, for example, to detecting a polymorphism in target sequences. More specifically this disclosure can identify a variant allele comprising a SNP compared to a wild type allele. This SNP can be associated with a particular disease status or diagnosis (e.g., with the diagnosis of sickle cell anemia, or diagnosis of a tumor or cancer). Additionally the SNP can be associated with a drug resistance or susceptibility. The isothermal amplification reaction methods and compositions described herein allow for the rapid detection of a target sequence and/or polymorphisms associated therein.

### EXAMPLES

### Example 1. Detection of BCR-T315I polymorphism using RPA and digestion with DdeI

Chronic myeloid leukemia (CML; also known as chronic myelogenous leukemia) is an uncommon cause of cancer-related mortality in the United States, with an estimated 6,660 new cases and 1,140 deaths anticipated in 2015. CML is characterized by the presence of the Philadelphia chromosome, a translocation between chromosomes 9 and 22 in humans, resulting in a fusion between the 5' end of the BCR (Breakpoint Cluster Region) gene and the 3' end of the ABL1 (ABL Proto-Oncogene 1, Non-Receptor Tyrosine Kinase) gene. Targeting the tyrosine kinase activity of BCR-ABL represents a very promising therapeutic strategy in CML and ABL1 kinase inhibitors, such as imatinib, have been developed as targeted therapies against BCR-ABL1 positive malignancies. Mechanistically, imatinib binds to the inactive form of BCR-ABL tyrosine kinase, preventing adenosine triphosphate (ATP) from binding. Presence of point mutations in BCR-ABL1 have been implicated as a mechanism for development of imatinib resistance. One such mutation, the T315I mutation is caused by a single cytosine to thymine (C to T) base pair substitution at position 944 of the Abl gene (codon '315' of the Abl protein) sequence resulting in amino acid (T)hreonine being substituted by (I)soleucine at that position, thus 'T315I'.

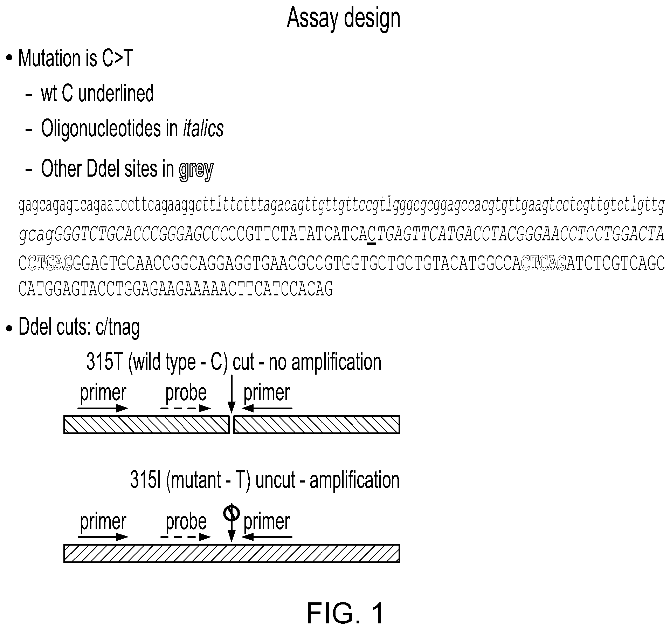

An allele specific RPA method was developed and optimized for determining the presence or absence of the C to T mutation of ABL1 (FIG 1). The assay was designed with a forward and a reverse primer for amplifying a target nucleic acid sequence overlapping with the C to T mutation of ABL1, the reverse primer having a portion complementary to the wild-type ABL1 "CTGAG" sequence. The primer sequence and probe sequence are identified in the below description.

The RPA reaction was performed in the presence of DdeI, a restriction endonuclease that recognizes the sequence C^TNA_G DdeI cuts products amplified from the ABL wildtype C/TNAG sequence, but does not cut products amplified from the ABL T315I mutation sequence (T/TNAG). Thus, the RPA amplification in presence of DdeI allows for enrichment and detection of mutant sequences.

### Materials and Methods:

TwistAmp^{®} exo pellets were resuspended with a master mix containing 29.5ul primer-free reaction buffer (PFRB), 4ul of 6uM Forward primer (CTTTTTCTTTAGACAGTTGTTTGTTCAGTTGGGAG), 4ul of 6uM Reverse primer (GGTAGTCCAGGAGGTTCCCGTAGGTCATGAACTCA) and 1ul of 6uM TwistAmp^{®} exo probe (tgaagtectcgttgtcttgttggc[MeOA]gGGG[T(ROX)](dSpacer)[T(BHQ-2)]GCACC[MeOC]GGGAGCC[*]C where * is a phosphothioate bond). Known copy numbers of human genomic DNA and/or variant synthetic BCR-ABL 315 DNA and either 10 units, or no units of the restriction endonuclease DdeI were added to different pellets to give a final volume of 47.5ul. Reactions were started by the addition of 2.5ul 280mM Magnesium acetate, which was added to the caps of the strip of tubes, which were closed and then simultaneously centrifuged to mix the magnesium acetate into the reactions. Reactions were mixed by vortexing, spun and placed in a Twista^{®} device that was preheated to 40°C. A 20 minute Twista^{®} run was started, with the strip removed, vortexed and centrifuged after 4 minutes to mix the reactions before they were replaced in the Twista^{®}.

### Results:

The restriction endonuclease DdeI cleaves any DNA containing the sequence CTNAG, where N can be any base. In this instance the wild type allele includes such a sequence and is cleaved, but the mutant form does not and so is not cleaved. The forward and reverse primers bind to complementary DNA and a strand displacing polymerase begins copying the complementary strand. If the DNA molecule being copied has been cleaved by the restriction endonuclease DdeI, the polymerase is unable to extend the strand it is synthesizing beyond this cleavage point. The DdeI restriction site lies in between the TwistAmp^{®} exo probe and opposing primer. The opposing primer should therefore not be able to synthesize the complementary DNA strand to the point where the TwistAmp^{®} exo probe can bind to it if the DdeI restriction endonuclease has cut the DNA. The primer targeting the same strand as the probe will also be unable to synthesize the complementary DNA strand to the opposing primer and so exponential amplification will not occur. When the TwistAmp^{®} exo probe binds to the complementary strand of any amplicon produced by the forward and reverse primers, it is cleaved by exonuclease III at the dSpacer (Tetrahydrofuran). This separates the Rox fluorophore from the Black Hole Quencher molecule. Every 20 seconds the Twista scans each tube, emitting light at 550nm and detecting it at 600nm. These two wavelengths are within the excitation and emissions spectra of Rox dye. When the fluorophore and quencher have been separated, the Twista^{®} excites the Rox dye and the Rox dye emits light at 600nm wavelength. If exponential amplification has occurred, then exponentially increasing numbers of probes bind to the complementary strands of the amplicon and are cleaved, releasing detectable free fluorophores and generating an exponential increase in fluorescence signal. If exponential amplification does not occur due to template cleavage by DdeI, then there is no exponential increase in fluorescence as the probe has insufficient complementary DNA to bind to and be cleaved.

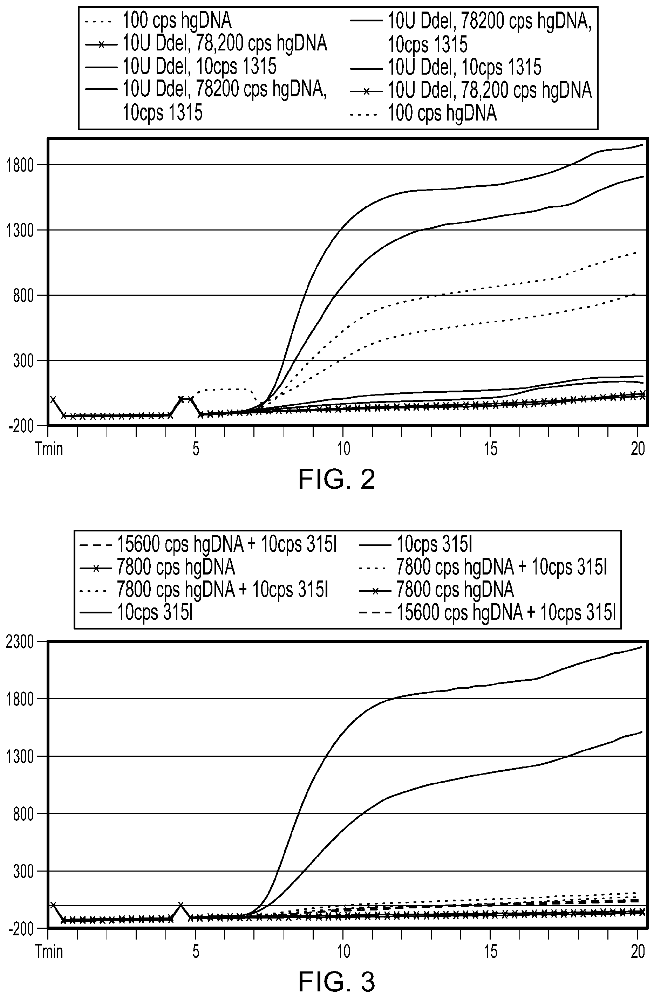

FIG. 2 is a graph summarizing example data generated a first enrichment experiment for BCR-ABL T315I RPA reactions in duplicate. Reactions represented by the dotted lines do not contain DdeI, just ~100 copies of wild type (wt) (315T) human genomic DNA (hgDNA). All other reactions contain 10 units of DdeI and either ~78,200 copies wt (315T) hgDNA showing no detectable amplification as expected; ~78,200 copies wt (315T) hgDNA plus 10 copies variant synthetic BCR-ABL 315 DNA, showing some amplification as expected; or 10 copies of variant synthetic BCR-ABL 315 DNA, showing good amplification as expected. The presence of an excess of wt (315T) hgDNA appears to be detrimental to the amplification of the variant (315I) allele, but signal is still detectable against background.

In the presence of DdeI, 273ng (~79,000 copies) of human genomic DNA and 10 copies of synthetic BCR-ABL 315 DNA generate a small, but detectable fluorescent signal. Without being bound to theory, it is believed that the reduced signal is due, in part, because the human genomic DNA and any amplicon generated from it is cut by DdeI before exponential amplification can occur, but this still consumes primers. The 10 copies of synthetic BCR-ABL 315 DNA do undergo exponential amplification, but the wild type human genomic DNA somehow inhibit this.

Thus 100 copies of uncut human genomic DNA and 10 copies of synthetic BCR-ABL 315 DNA show detectable levels of fluorescence as the amplicon is exponentially copied by the primers, allowing an exponential increase in the cleavage of the TwistAmp^{®} exo probe (see FIG. 2). In the presence of DdeI, 273ng (~79,000 copies) of human genomic DNA do not generate a detectable fluorescent signal, presumably because the human genomic DNA and any amplicon generated is cut by DdeI before exponential amplification can occur.

FIG 3 is a graph summarizing example data generated in a second enrichment experiment for BCR-ABL T315I RPA reactions in duplicate. All reactions contain 10Units DdeI. Reactions containing only wt human genomic DNA (hgDNA) are flat, as expected. Reactions represented by the dashed and the dotted lines contain 10 copies of variant synthetic BCR-ABL 315 DNA and 15,600 or 7800 copies of wt (315T) hgDNA DNA respectively, showing that the variant amplifies even against a background of wt hgDNA, although signal is clearly inhibited when compared to reactions containing only 10 copies of variant synthetic BCR-ABL 315 DNA, that show good amplification (solid lines).

When 10 copies of synthetic BCR-ABL 315 DNA and either 7,800 or 15,600 copies of human genomic DNA are present, they generate a small, but detectable fluorescent signal. This is presumably because the human genomic DNA and any amplicon generated from it is cut by DdeI before exponential amplification can occur, but this still consumes primers. The 10 copies of synthetic BCR-ABL 315 DNA do undergo exponential amplification, but the wild type human genomic DNA somehow inhibit this. This inhibition hypothesis is backed up as it is noticeable that the signal is stronger when there is less human genomic DNA present (7,800 copies versus 15,600 copies). Thus 10 copies of synthetic BCR-ABL 315 DNA show detectable levels of fluorescence as the amplicon is exponentially copied by the primers, allowing an exponential increase in the cleavage of the TwistAmp^{®} exo probe. In the presence of DdeI, 7,800 copies of human genomic DNA do not generate a detectable fluorescent signal, presumably because the human genomic DNA and any amplicon generated is cut by DdeI before exponential amplification can occur.

### Example 2. Detection of rs334 polymorphism using RPA and digestion with Hpy 166II and DdeI

Beta thalassemias (β thalassemias) are a group of inherited blood disorders. They are caused by reduced or absent synthesis of the beta subunit of hemoglobin that result in variable outcomes ranging from severe anemia to clinically asymptomatic individuals. One form of β thalassemias is commonly referred to as sickle cell anemia. The disease is caused by a single-base polymorphism, SNP rs334, in the beta subunit (β-globin) hemoglobin gene. rs334(A) encodes the normal Hb A form of (adult) hemoglobin. rs334(T) encodes the sickling form of hemoglobin, Hb S. Thus, the "normal" allele is A, whereas the mutated one is T. Only individuals homozygous for this allele, in other words having the rs334(T;T) genotype, will have sickle cell anemia.

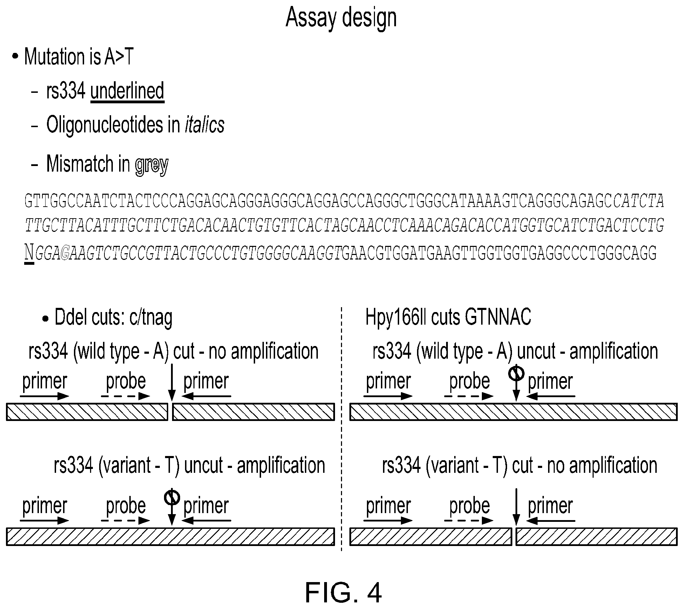

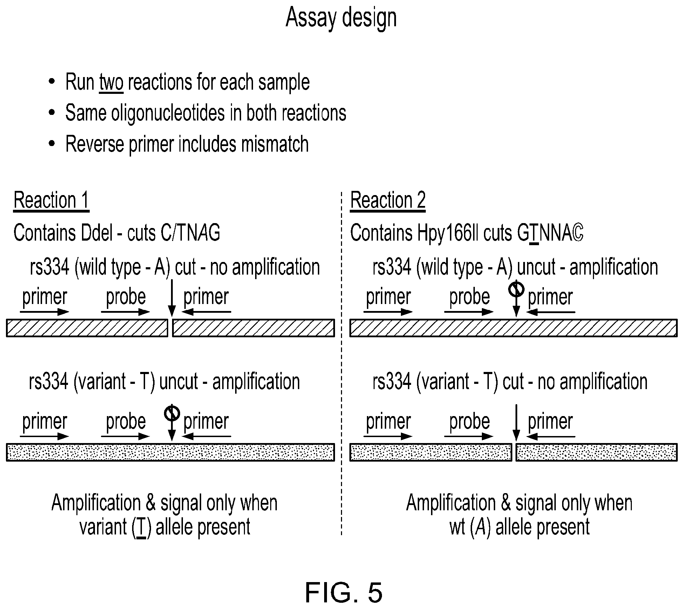

An allele specific RPA method was developed and optimized for determining the presences or absence of the rs334(A) to rs334(T) polymorphism of β-globulin using restriction endonuclease Ddel and Hpy166II (FIG 4 and 5).

Wild type (A) contains DdeI restriction endonuclease target sequence (CTNAG).
ATCTGACTCCTG**A**GGAGAAGTCTGCCGTTACTGCCCTGTGGGGCAAGGT (SEQ ID NO: 1)

The variant (T) allele differs from Hpy 166II restriction endonuclease target sequence (GTNNAC) by 1 base:
ATCTGACTCCTG**T**GG**A**GAAGTCTGCCGTTACTGCCCTGTGGGGCAAGGT (SEQ ID NO: 2)

Amplification with a mismatched primer can produce amplicon that contains an Hpy 166I1 cut site when Variant (T) allele is present, but not when wt (A) allele is present. Variant amplicon (C introduced by primer mismatch) - can be cut by Hpy 166II:
ATCTGACTCCTG**T**GGA***C***AAGTCTGCCGTTACTGCCCTGTGGGGCAAGGT (SEQ ID NO: 3)
Wt amplicon (C introduced by primer mismatch) - cannot be cut by Hpy 166II ATCTGACTCCTG*A*GGA*C*AAGTCTGCCGTTACTGCCCTGTGGGGCAAGGT (SEQ ID NO: 4)

### Materials and Methods:

Two RPA reactions were performed for each sample (see FIG 5). The same oligonucleotides were present in each reaction, the first reaction (Reaction 1) contains DdeI and the second reaction (Reaction 2) contains Hpy 166II. For the first reaction, amplification and signal generation occur only when the variant (T) allele is present. For the second reaction, amplification and signal generation occur only when the wildtype (A) allele is present.

TwistAmp^{®} exo pellets were resuspended with a master mix containing 29.5ul PFRB buffer, 4ul of 6uM Forward primer (CATCTATTGCTTACATTTGCTTCTGACACAAC)(SEQ ID NO: 5), 4ul of 6uM Reverse primer (ACCTTGCCCCACAGGGCAGTAACGGCAGACTTGTC) (SEQ ID NO: 6) and 1ul of 6uM TwistAmp^{®} exo probe (TGTTCACTAGCAACCTCAAACAGACACCA[T(ROX)](dSpacer)G[T(BHQ-2)]GCATCTGACTCC[*]T where * is a Phosphothioate bond)(SEQ ID NO: 7). One thousand copies of wild type or variant rs334 synthetic DNA template and either 10 units of the restriction endonuclease DdeI or 1 unit of the restriction endonuclease Hpy166II were added to different pellets to give a final volume of 47.5ul. Reactions were started by the addition of 2.5ul 280mM Magnesium acetate, which was added to the caps of the strip of tubes, which were closed and then the magnesium acetate simultaneously centrifuged into the reactions. Reactions were mixed by vortexing, spun and placed in a Twista^{®} device that was preheated to 39°C. A 20 minute Twista^{®} run was started, with the strip removed, vortexed and centrifuged after 4 minutes to mix the reactions before they were replaced in the Twista^{®}.

### Results:

The restriction endonuclease DdeI cleaves any DNA containing the sequence CTNAG, where N can be any base. In this instance the wild type (A) allele includes such a sequence and is cleaved, but the variant (T) form does not and so is not cleaved. The restriction endonuclease Hpy 166II cleaves any DNA containing the sequence GTNNAC. This sequence does not occur in either allele, but can be introduced into the variant (T) amplicon if a mismatched primer is used. Thus DdeI cleaves wild type genomic DNA and wild type amplicon, whilst Hpy 166II only cleaves variant amplicon, but not variant genomic DNA. The forward and reverse primers bind to complementary DNA and a strand displacing polymerase begins copying the complementary strand. If the DNA molecule being copied has been cleaved by the restriction endonuclease DdeI or Hpy166II, the polymerase is unable to extend the strand it is synthesizing beyond this cleavage point. The DdeI and Hpy 166II restriction sites lie in between the regions where the TwistAmp^{®} exo probe and opposing primer hybridize. The opposing primer should therefore not be able to synthesize the complementary DNA strand to the point where the TwistAmp^{®} exo probe can bind to it if DdeI or Hpy166II has cut the DNA. The primer targeting the same strand as the probe will also be unable to synthesize the complementary DNA strand to the opposing primer and so exponential amplification will not occur. When the TwistAmp^{®} exo probe binds to the complementary strand of any amplicon produced by the forward and reverse primers, it is cleaved by exonuclease III at the dSpacer (Tetrahydrofuran). This separates the Rox fluorophore from the Black Hole Quencher^{®} molecule. Every 20 seconds the Twista^{®} scans each tube, emitting light at 550nm and detecting it at 600nm. These two wavelengths are within the excitation and emissions spectra of Rox dye. When the fluorophore and quencher have been separated, the Twista^{®} excites the Rox dye and the Rox dye emits light at 600nm wavelength. If exponential amplification has occurred, then exponentially increasing numbers of probes bind to the complementary strands of the amplicon and are cleaved, releasing detectable free fluorophores and generating an exponential increase in fluorescence signal. If exponential amplification does not occur due to template and/or amplicon cleavage by DdeI or Hpy166II, then there is no exponential increase in fluorescence as the probe has insufficient complementary DNA to bind to and be cleaved. Thus 1000 copies of wild-type synthetic DNA template show detectable levels of fluorescence in the presence of 1 unit of Hpy166II as the synthetic DNA template and amplicon generated is not cleaved and can be exponentially copied by the primers, allowing an exponential increase in the cleavage of the TwistAmp^{®} exo probe. However 1000 copies of wild type synthetic DNA template in the presence of 10 units of DdeI show no detectable signal as they are cleaved before exponential amplification can occur.

For 1000 copies of the variant allele, the signal in the presence of 10 units of DdeI (which should not cleave it), is visible much earlier than when 1 unit of Hpy 166II is present (which should cleave any amplicon, but not the original template). The fact that there is amplification at all in the presence of 1 unit of Hpy 166II suggests that there is insufficient enzyme to cleave the amplicon as it is generated before complementary DNA to the probe can be synthesized. As Hpy166II is supplied as a glycerol suspension at a low concentration it is difficult to add more without seeing inhibition of the amplification reaction due to the presence of glycerol per se.

FIG 6 is a graph summarizing data generated by running the two different rs334 reactions in duplicate. Reactions containing DdeI are shown as solid lines, reactions containing Hpy166II are shown as dashed lines. The DdeI reactions containing only wt (AA- solid lines) template show no detectable amplification as expected, while those containing only variant (TT -lines with X's) template show detectable amplification after 6-7 minutes. The Hpy 166II reactions containing only variant (TT - dotted lines) template show some amplification, but much less than the equivalent copy number of wt (AA- dashed lines) templates. This suggests that Hpy166II cutting of perfect match template is <100%, but >0%.

FIG 7 is a diagram showing, in an ideal scenario, cleavage and suppression of amplification of perfect match template is complete for both restriction endonuclease. In this case when plotting time to signal (either a predetermined threshold, or rate of signal change) in the wild type reaction and the variant reaction one will see a distribution of data points similar to that shown on the left panel (Ideal scenario), allowing for clustering by genotype (wt/wt, wt/var, var/var). However, it is still possible to differentiate genotypes if suppression of amplification is not 100% by comparing the signal in reaction 1 to that in reaction 2. The scenario on the right (Actual Results) shows an illustration of this where one restriction endonuclease, DdeI cuts perfect match template completely, but the other, Hpy166II does not. The three possible genotypes can still be distinguished by comparing the ratio of the DdeI reaction time to signal with the Hpy 166II reaction time to signal (assuming that roughly equivalent amounts of template are added to both reactions).

### Example 3. Genotyping using CRISPR-RPA genotyping

The compositions and methods disclosed herein can also be used for diagnosing and detecting polymorphisms that occur at any target nucleic acid sequence using an engineered sequence-specific nuclease. An example of such a sequence-specific nuclease is the CRISPR technology. The CRISPR system works by recruiting a nuclease (e.g., the Cas enzyme) to a specific DNA target using a short RNA molecule. It is this short RNA molecule that can be designed to be complementary to a particular nucleic acid target.

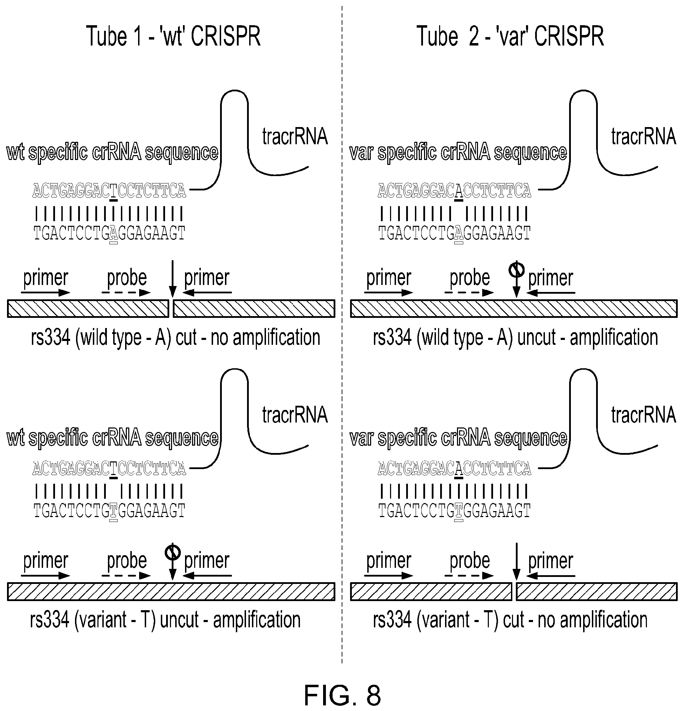

FIG 8 is a schematic demonstrating a genotyping system using CRISPR-RPA for the detection of an rs334 polymorphism. Here, the short RNA (crRNA or CRISPR RNA) is designed to guide a nuclease, which is complexed with the crRNA and the separate tracrRNA (trans-activating crRNA), to a specific nucleic acid sequence which will then be cleaved.

In tube 1, the crRNA is designed to be complementary to and to recognize the wild type sequence of TGAGG When the target nucleic acid is the wild type sequence, then the CRISPR-Cas system will direct the Cas enzyme to cleave at the wild type sequence and there will be no amplification of the target region. If, however, the wt sequence contains a polymorphism (i.e. variant-T sequence of TGTGG) within the sequence that is complementary to the crRNA then the crRNA will not recognize the sequence. If the crRNA is not complementary to the polymorphism containing sequence, then the target sequence will not be cleaved and amplification of the target sequence will occur.

In tube 2, the crRNA is designed to recognize the variant containing the polymorphism, i.e., to be complementary to the variant sequence TGTGG Therefore, the CRISPR-system will direct the nuclease to the polymorphism containing sequence, the sequence will be cleaved, and there will be no amplification. The wild type sequence will not be recognized by the sequence-specific nuclease and so there will be amplification of the wild type sequence.

## Claims

1. An isothermal amplification reaction method of determining the genotype of a target allele in a sample comprising double-stranded nucleic acids, wherein the target allele comprises a wild-type sequence or a variant sequence that comprises one or more single nucleotide polymorphisms (SNPs) compared to the wild-type sequence, comprising:
(a) contacting the target allele in a first reaction with a first primer, a second primer, a recombinase, a polymerase, and a first agent capable of cleaving double-stranded nucleic acid at a first specific cleavage sequence, wherein said first primer and said second primer are a forward and reverse primer pair and wherein the first specific cleavage sequence is located (i) within the wild-type sequence between the portions of the target nucleic acid sequence complementary to the first and second primers; or (ii) within or overlaps with portions of the wild-type sequence complementary to the first and second primers;
(b) performing amplification by extending the primers along the sequence of the target allele to produce amplified replication products of the target allele;
(c) cleaving the wild-type sequence and the amplified products with the first agent;
(d) repeating amplification such that the nucleic acid molecules that comprise a sequence that is different from the first specific cleavage sequence at one or more positions are amplified at a higher rate than amplified products that comprise the first specific cleavage sequence;
(e) detecting the amplified products, wherein the amplified products are detected using fluorescence, phase contrast microscopy, luminescent detection, spectral (color) detection, magnetic detection, radioisotopic detection and/or electrochemical detection;
(f) contacting the target allele in a second reaction with a third primer, a fourth primer, a recombinase, a polymerase, and a second agent capable of cleaving double-stranded nucleic acid at a second specific cleavage sequence, wherein said third primer and said fourth primer are a forward and reverse primer pair, wherein the second specific cleavage sequence is different from the wild-type sequence at two or more SNPs, wherein the sequence of the third primer is complementary to that of the target allele, wherein the variant sequence comprises a first SNP of the two or more SNPs and wherein the fourth primer comprises a second SNP of the two or more SNPs and comprises at least part of the second specific cleavage sequence, such that the fourth primer introduces the second specific cleavage sequence when amplified with the variant sequence,
(g) performing amplification as in (b), wherein the extension of the third primer comprises an identical replication of the target nucleic acid, and the extension of the fourth primer comprises the sequence of the fourth primer;
(h) cleaving the amplified replication products of (g) with the second agent;
(i) repeating amplification such that the amplified replication products with a sequence that is different from the second specific cleavage sequence at one or more positions are amplified at a higher rate than the amplified replication products with the second specific cleavage sequence;
(j) detecting the amplified products, wherein the amplified products are detected using fluorescence, phase contrast microscopy, luminescent detection, spectral (color) detection, magnetic detection, radioisotopic detection and/or electrochemical detection; and
(k) comparing the detection of (e) to the detection of (j).

2. The method of claim 1, wherein:
i) the second amplified replication product comprises the second specific cleavage sequence; or
ii) the amplified products are detected as in (e) and (j) at a multitude of times.

3. The method of any of claims 1 to 2, wherein the target allele of (a) and (f) is further contacted with a probe labeled with a detectable label, optionally wherein:
i) the probe comprises an oligonucleotide complimentary to a portion of the target nucleic acid sequence at a position that is in between the sequences where the first and the second primers bind the target nucleic acid; or
ii) the detectable label is selected from a group consisting of an enzyme, an enzyme substrate, a coenzyme, an enzyme inhibitor, a fluorescent marker, a quencher, a chromophore, a magnetic particle or bead, a redox sensitive moiety, a luminescent marker, a radioisotope, and members of binding pairs; or
iii) the label comprises a fluorescent marker that is selected from the group consisting of fluorescein, FAM, TAMRA (tetramethylrhodamine) and Texas Red^{™}.

## Patentansprüche

1. Ein isothermes Amplifikationsreaktionsverfahren zum Bestimmen des Genotyps eines Zielallels in einer Probe, die doppelsträngige Nukleinsäuren umfasst, wobei das Zielallel eine Wildtypsequenz oder eine variante Sequenz umfasst, die ein oder mehrere Einzelnukleotidpolymorphismen (SNPs) verglichen mit der Wildtypsequenz umfasst, das Folgendes umfasst:
(a) In-Kontakt-Bringen des Zielallels in einer ersten Reaktion mit einem ersten Primer, einem zweiten Primer, einer Rekombinase, einer Polymerase und einem ersten Mittel, das in der Lage ist, doppelsträngige Nukleinsäure an einer ersten spezifischen Spaltsequenz zu spalten, wobei der genannte erste Primer und der genannte zweite Primer ein Vorwärts- und Rückwärts-Primerpaar sind und wobei sich die erste spezifische Spaltsequenz befindet: (i) innerhalb der Wildtypsequenz zwischen den Anteilen der Zielnukleinsäuresequenz, die komplementär zu dem ersten und zweiten Primer sind; oder (ii) innerhalb oder überlappend mit Anteilen der Wildtypsequenz, die komplementär zu dem ersten und zweiten Primer sind;
(b) Durchführen einer Amplifikation durch Verlängern der Primer entlang der Sequenz des Zielallels, um amplifizierte Replikationsprodukte des Zielallels herzustellen;
(c) Spalten der Wildtypsequenz und der amplifizierten Produkte mit dem ersten Mittel;
(d) Wiederholen der Amplifikation, sodass die Nukleinsäuremoleküle, die eine Sequenz umfassen, die sich von der ersten spezifischen Spaltsequenz an einer oder mehreren Positionen unterscheidet, mit einer höheren Rate amplifiziert werden als amplifizierte Produkte, die die erste spezifische Spaltsequenz umfassen;
(e) Nachweisen der amplifizierten Produkte, wobei die amplifizierten Produkte unter Verwendung von Fluoreszenz, Phasenkontrastmikroskopie, Lumineszenznachweis, Spektral(farb)nachweis, magnetischem Nachweis, Radioisotopennachweis und/oder elektrochemischem Nachweis nachgewiesen werden;
(f) In-Kontakt-Bringen des Zielallels in einer zweiten Reaktion mit einem dritten Primer, einem vierten Primer, einer Rekombinase, einer Polymerase und einem zweiten Mittel, das in der Lage ist, doppelsträngige Nukleinsäure an einer zweiten spezifischen Spaltsequenz zu spalten, wobei der genannte dritte Primer und der genannte vierte Primer ein Vorwärts- und Rückwärts-Primerpaar sind, wobei sich die zweite spezifische Spaltsequenz an zwei oder mehreren SNPs von der Wildtypsequenz unterscheidet, wobei die Sequenz des dritten Primers zu der des Zielallels komplementär ist, wobei die variante Sequenz einen ersten SNP von den zwei oder mehreren SNPs umfasst und wobei der vierte Primer einen zweiten SNP von den zwei oder mehreren SNPs umfasst und mindestens Teil der zweiten spezifischen Spaltsequenz umfasst, sodass der vierte Primer die zweite spezifische Spaltsequenz einführt, wenn mit der varianten Sequenz amplifiziert wird,
(g) Durchführen einer Amplifikation wie in (b), wobei die Verlängerung des dritten Primers eine identische Replikation der Zielnukleinsäure umfasst und die Verlängerung des vierten Primers die Sequenz des vierten Primers umfasst;
(h) Spalten der amplifizierten Replikationsprodukte von (g) mit dem zweiten Mittel;
(i) Wiederholen der Amplifikation, sodass die amplifizierten Replikationsprodukte mit einer Sequenz, die sich von der zweiten spezifischen Spaltsequenz an einer oder mehreren Positionen unterscheidet, mit einer höheren Rate amplifiziert werden als die amplifizierten Replikationsprodukte mit der zweiten spezifischen Spaltsequenz;
(j) Nachweisen der amplifizierten Produkte, wobei die amplifizierten Produkte unter Verwendung von Fluoreszenz, Phasenkontrastmikroskopie, Lumineszenznachweis, Spektral(farb)nachweis, magnetischem Nachweis, Radioisotopennachweis und/oder elektrochemischem Nachweis nachgewiesen werden; und
(k) Vergleichen des Nachweises von (e) mit dem Nachweis von (j).

2. Verfahren nach Anspruch 1, wobei:
i) das zweite amplifizierte Replikationsprodukt die zweite spezifische Spaltsequenz umfasst; oder
ii) die amplifizierten Produkte wie in (e) und (j) zu mehreren Zeitpunkten nachgewiesen werden.

3. Verfahren nach einem der Ansprüche 1 bis 2, wobei das Zielallel von (a) und (f) ferner mit einer Sonde in Kontakt gebracht wird, die mit einer nachweisbaren Markierung markiert ist, wobei optional:
i) die Sonde ein Oligonukleotid umfasst, das komplementär zu einem Anteil der Zielnukleinsäuresequenz an einer Position ist, die zwischen den Sequenzen liegt, wo der erste und der zweite Primer die Zielnukleinsäure binden; oder
ii) die nachweisbare Markierung aus der Gruppe ausgewählt ist, die aus einem Enzym, einem Enzymsubstrat, einem Coenzym, einem Enzymhemmer, einem fluoreszierenden Marker, einem Quencher, einem Chromophor, einem Magnetteilchen oder Kügelchen, einem redoxempfindlichen Teil, einem lumineszierenden Marker, einem Radioisotop und Glieder von Bindungspaaren besteht; oder
iii) die Markierung einen fluoreszierenden Marker umfasst, der aus der Gruppe ausgewählt ist, die aus Fluorescein, FAM, TAMRA (Tetramethylrhodamin) und Texas Red^{™} besteht.

## Revendications

1. Procédé de réaction d'amplification isotherme permettant de déterminer le génotype d'un allèle cible dans un échantillon comprenant des acides nucléiques double brin, l'allèle cible comprenant une séquence de type sauvage ou une séquence variante qui comprend un ou plusieurs polymorphismes mononucléotidiques (SNP) comparativement à la séquence de type sauvage, comprenant :
(a) une mise en contact de l'allèle cible dans une première réaction avec une première amorce, une deuxième amorce, une recombinase, une polymérase, et un premier agent capable de cliver un acide nucléique double brin au niveau d'une première séquence de clivage spécifique, ladite première amorce et ladite deuxième amorce étant une paire d'amorces sens et antisens et la première séquence de clivage spécifique étant située (i) dans la séquence de type sauvage entre les parties de la séquence d'acide nucléique cible complémentaires aux première et deuxième amorces ; ou (ii) dans ou chevauchant des parties de la séquence de type sauvage complémentaires aux première et deuxième amorces ;
(b) la réalisation d'une amplification par extension des amorces le long de la séquence de l'allèle cible pour produire des produits de réplication amplifiés de l'allèle cible ;
(c) un clivage de la séquence de type sauvage et des produits amplifiés avec le premier agent ;
(d) une répétition de l'amplification de telle sorte que les molécules d'acide nucléique qui comprennent une séquence qui est différente de la première séquence de clivage spécifique à une ou plusieurs positions soient amplifiées à un taux plus élevé que les produits amplifiés qui comprennent la première séquence de clivage spécifique ;
(e) une détection des produits amplifiés, les produits amplifiés étant détectés par fluorescence, microscopie à contraste de phase, détection par luminescence, détection spectrale (couleur), détection magnétique, détection radio-isotopique et/ou détection électrochimique ;
(f) une mise en contact de l'allèle cible dans une deuxième réaction avec une troisième amorce, une quatrième amorce, une recombinase, une polymérase et un deuxième agent capable de cliver un acide nucléique double brin au niveau d'une deuxième séquence de clivage spécifique, ladite troisième amorce et ladite quatrième amorce étant une paire d'amorces sens et antisens, la deuxième séquence de clivage spécifique étant différente de la séquence de type sauvage au niveau de deux SNP ou plus, la séquence de la troisième amorce étant complémentaire à celle de l'allèle cible, la séquence variante comprenant un premier SNP des deux SNP ou plus et la quatrième amorce comprenant un deuxième SNP des deux SNP ou plus et comprenant au moins une partie de la deuxième séquence de clivage spécifique, de telle sorte que la quatrième amorce introduise la deuxième séquence de clivage spécifique lorsqu'elle est amplifiée avec la séquence variante ;
(g) la réalisation d'une amplification comme à l'étape (b), dans laquelle l'extension de la troisième amorce comprend une réplication identique de l'acide nucléique cible, et l'extension de la quatrième amorce comprend la séquence de la quatrième amorce ;
(h) un clivage des produits de réplication amplifiés de l'étape (g) avec le deuxième agent ;
(i) une répétition de l'amplification de telle sorte que les produits de réplication amplifiés ayant une séquence qui est différente de la deuxième séquence de clivage spécifique à une ou plusieurs positions soient amplifiés à un taux plus élevé que les produits de réplication amplifiés ayant la deuxième séquence de clivage spécifique ;
(j) une détection des produits amplifiés, les produits amplifiés étant détectés par fluorescence, microscopie à contraste de phase, détection par luminescence, détection spectrale (couleur), détection magnétique, détection radio-isotopique et/ou détection électrochimique ; et
(k) une comparaison de la détection à l'étape (e) avec la détection à l'étape (j).

2. Procédé selon la revendication 1, dans lequel :
i) le deuxième produit de réplication amplifié comprend la deuxième séquence de clivage spécifique ; ou
ii) les produits amplifiés sont détectés comme à l'étape (e) et à l'étape (j) une multitude de fois.

3. Procédé selon l'une quelconque des revendications 1 ou 2, dans lequel l'allèle cible de l'étape (a) et de l'étape (f) est en outre mis en contact avec une sonde marquée à l'aide d'un marqueur détectable, optionnellement dans lequel :
i) la sonde comprend un oligonucléotide complémentaire à une partie de la séquence d'acide nucléique cible à une position qui est située entre les séquences où les première et deuxième amorces se lient à l'acide nucléique cible ; ou
ii) le marqueur détectable est sélectionné dans un groupe constitué d'une enzyme, d'un substrat enzymatique, d'une coenzyme, d'un inhibiteur enzymatique, d'un marqueur fluorescent, d'un quencher, d'un chromophore, d'une particule ou d'une bille magnétique, d'une fraction sensible à l'oxydoréduction, d'un marqueur luminescent, d'un radio-isotope et d'éléments de paires de liaison ; ou
iii) le marqueur comprend un marqueur fluorescent qui est sélectionné dans le groupe constitué de la fluorescéine, de FAM, de TAMRA (tétraméthylrhodamine) et du Texas Red^{™}.
